(19) Europäisches Patentamt
European Patent Office
Office européen des brevets

(11) **EP 3 923 491 A1**

(12) **EUROPEAN PATENT APPLICATION**

(43) Date of publication:
**15.12.2021 Bulletin 2021/50**

(51) Int Cl.:
*H04B 10/516* (2013.01)      *G01B 9/02* (2006.01)
*G01J 3/02* (2006.01)

(21) Application number: **20179849.3**

(22) Date of filing: **12.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nokia Technologies Oy
02610 Espoo (FI)**

(72) Inventors:
• **YUAN, Xin
  New Providence, NJ New Jersey 07974 (US)**
• **QIAO, Mu
  Summit, NJ New Jersey 07901 (US)**
• **LIU, Xuan
  Berkeley Heights, NJ New Jersey 07922 (US)**

(74) Representative: **Swindell & Pearson Limited
48 Friar Gate
Derby DE1 1GY (GB)**

(54) **APPARATUS, SYSTEMS AND METHODS FOR COMPRESSIVE SENSING**

(57)   An apparatus comprising:
a detector configured to detect, cumulatively during an exposure period, spatially modulated light; and
modulation means for applying multiple different effective spatial modulations to received light, during the exposure period, wherein a different effective spatial modulation is applied to received light in dependence upon a time during the exposure period of the detector and a frequency of the light, to produce spatially modulated light for detection by the detector.

FIG. 1

**Description**

TECHNOLOGICAL FIELD

Examples of the disclosure relate to apparatus, systems and methods for enabling compressive sensing.

BACKGROUND

According to the theory of compressive sensing, traditional sampling is replaced by measurements of inner products with random vectors.

Light modulated by reflection from or transmission through an object, when detected directly by a two-dimensional pixelated detector, is an oversampled field that has a sparser representation in some domain. As a consequence, detecting spectrally dispersed coded fields (sparse incoherent fields rather than the whole field) can capture sufficient information to characterise the object. For example, spectral images of the object can be determined from the detected spectrally dispersed coded fields.

BRIEF SUMMARY

According to various, but not necessarily all, embodiments of the invention there is provided an apparatus comprising:

a detector configured to detect, cumulatively during an exposure period, spatially modulated light; and modulation means for applying multiple different effective spatial modulations to received light during an exposure period of a detector, wherein a different effective spatial modulation is applied to received light in dependence upon a time during the exposure period and a frequency of the light, to produce spatially modulated light for detection by the detector.

The apparatus is able to detect light at a higher speed than the detector. It is therefore possible to obtain high speed detection with a lower speed detector.

In some but not necessarily all examples, the different effective spatial modulation is configured to cause modulation by different spatial modulation patterns, at the detector, of the received light, wherein different spatial patterns are used for different time divisions within the exposure period and wherein, within each time division, different spatial patterns are used for distinct frequency ranges.

In some but not necessarily all examples, dispersing means is configured to enable the use, within each time division, of different spatial patterns for distinct frequency ranges.

In some but not necessarily all examples, the modulation means is configured to apply a different spatial modulation pattern to the received light during each time division of the exposure period and is configured to apply, subsequently, different spatial offsets to the light in dependence upon frequency.

In some but not necessarily all examples, the modulation means comprises:

spatial modulating means for spatially modulating a beam of light to produce a modulated beam of light; dispersing means for dispersing the modulated beam of light to produce a spatially modulated and dispersed beam of light; and means for causing a time-variation of the spatial modulating means and/or the dispersing means during the exposure period.

In some but not necessarily all examples, the means for causing a time-variation of the spatial modulating means during the exposure period causes a time-variation of a spatial modulation pattern applied during the exposure period by the spatial modulating means.

In some but not necessarily all examples, the spatial modulation is randomized, pixelated modulation over a two-dimensional area.

In some but not necessarily all examples, the spatial modulation is pixelated modulation over a two-dimensional area, and the pixelated modulation comprises pixels arranged in rows and columns that are parallel to rows and columns of pixels of the detector when projected onto the detector.

In some but not necessarily all examples, the spatial modulating means comprises a two-dimensional spatially coded aperture comprising at least a first plurality of portions, having a first transparency, and at least a second plurality of portions, having a second different transparency, wherein the first plurality of portions and the second plurality of portions are spatially distributed in two dimensions.

In some but not necessarily all examples, the spatial modulating means comprises a spatially coded aperture that has a time variable pattern or has a fixed pattern and is movable.

In some but not necessarily all examples, the means for causing a time-variation of the dispersing means during the exposure period causes a time variation of a position of the dispersion means during the exposure period.

In some but not necessarily all examples, the dispersing means comprises one or more refractive elements or one or more diffractive elements.

In some but not necessarily all examples, the apparatus comprises a double path interferometer comprising a sample path for an object and a reference path; means for superposing the sample path and reference path during measurement to create a beam of light for spatial modulation and detection.

In some but not necessarily all examples, there is provided a system comprising the apparatus, wherein the system or apparatus further comprises processing means for processing output of the detector to obtain a three dimensional image of at least part of an object, wherein an input beam of light for spatial modulation and detection by the apparatus is obtained from an optical coherence tomography arrangement and comprises light reflected from the object; and means for providing a three dimensional image of at least part of the object from the electrical output signals.

According to various, but not necessarily all, embodiments of the invention there is provided a method comprising:

applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector; and

detecting, during the exposure period, the spatially modulated light.

According to various, but not necessarily all, embodiments of the invention there is provided a computer program that when run by a processor enables the processor to control: applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector.

According to various, but not necessarily all, embodiments of the invention there is provided an apparatus comprising:

at least one processor; and

at least one memory including computer program code

the at least one memory and the computer program code configured to, with the at least one processor, cause the apparatus at least to perform:

applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector.

According to various, but not necessarily all, embodiments of the invention there is provided an apparatus comprising: an apparatus comprising:

a detector configured to detect, cumulatively during an exposure period, spatially modulated light; and

a modulator configured to apply multiple different effective spatial modulations to received light during the exposure period, wherein a different effective spatial modulation is applied to received light in dependence upon a time during the exposure period and a frequency of the light, to produce spatially modulated light for detection by the detector.

According to various, but not necessarily all, embodiments of the invention there is provided an apparatus comprising:

spatial modulating means for spatially modulating a beam of light to produce a modulated beam of light;

dispersing means for dispersing the modulated beam of light to produce a spatially modulated and dispersed beam of light;

a detector configured to detect, cumulatively during an exposure period, the spatially modulated and dispersed beam of light; and

means for causing a time-variation of the spatial modulating means and/or the dispersing means during the exposure period.

According to various, but not necessarily all, embodiments of the invention there is provided an apparatus comprising:

a spatial modulator (e.g. a spatially coded mask) configured to apply a time-varying spatial modulation, during an exposure period of a detector, to a beam of light to produce a time-varying modulated beam of light;
a dispersing element configured to disperse the modulated beam of light to produce a time-varying spatially modulated and dispersed beam of light; and
a detector configured to detect, cumulatively during the exposure period of the detector, the time-varying spatially modulated and dispersed beam of light.

According to various, but not necessarily all, embodiments of the invention there is provided examples as claimed in the appended claims.

BRIEF DESCRIPTION

For a better understanding of various examples that are useful for understanding the detailed description, reference will now be made by way of example only to the accompanying drawings in which:

Fig. 1 shows an example of the apparatus;
Fig. 2 shows an example of a compressive sensing principle of examples of the disclosure;
Fig. 3 illustrates an example of an optical coherence tomography arrangement for measurement; and
Fig. 4 illustrates an example of a method.

DETAILED DESCRIPTION

Examples of the disclosure relate to an apparatus 1 comprising:

a detector 7 configured to detect, cumulatively during an exposure period, spatially modulated light 13; and
modulation means 2 for applying multiple different effective spatial modulations to received light 9 during the exposure period, wherein a different effective spatial modulation is applied to received light 9 in dependence upon a time during the exposure period and a frequency of the light, to produce spatially modulated light 13 for detection by the detector 7.

In some but not necessarily all examples, the beam of light 9 can be provided by an optical coherence tomography (OCT) arrangement, which acts as the source 17.

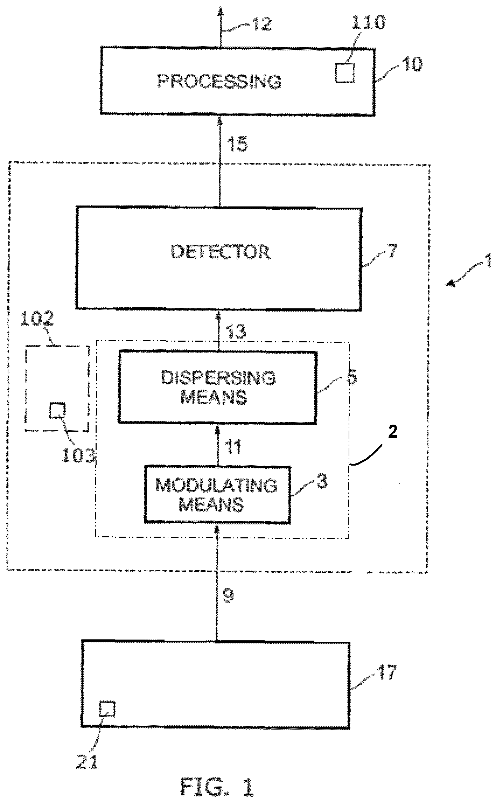

Fig. 1 schematically illustrates an example apparatus 1 for compressive sensing. The example apparatus 1 comprises:

a detector 7 configured to detect, cumulatively during an exposure period, spatially modulated light 13; and
modulation means 2 for applying multiple different effective spatial modulations to received light during an exposure period of a detector, wherein a different effective spatial modulation is applied to received light 9 in dependence upon a time during the exposure period and a frequency of the light, to produce spatially modulated light 13 for detection by the detector 7.

The exposure period can be logically time-divided into different sequential time divisions. The time-divisions are non-overlapping. In some examples the time-divisions are contiguous. In some examples the time-divisions are of equal duration. In some examples the time divisions are the same for different exposure periods.
The received light 9 comprises light within a band of frequencies. This band of frequencies can be logically frequency-divided into different frequency ranges. The frequency ranges can be non-overlapping. In some examples the frequency ranges are contiguous. In some examples the frequency ranges are not of equal size.
Each combination of frequency range and time division can be considered a time-frequency tile in time-frequency space. A different spatial modulation pattern can be used for light from different time-frequency tiles.
While it is useful to consider the logical division of the time-frequency space into tiles, it should be appreciated that the physical division can be performed by analogue or digital means. Where analogue means are used, for example dispersion means, the edges of the time-frequency tiles, particularly in the frequency domain, are not necessarily sharp.
The different effective spatial modulation can cause modulation, by different spatial modulation patterns, at the detector 7, of the received light 9, wherein different spatial patterns are used for different time divisions within the exposure period and wherein, within each time division, different spatial patterns are used for distinct frequency ranges.

For example, a different effective spatial modulation pattern $\acute{Z}^l_k$ can be applied for each different combination (*l*, *k*) where *l* is an index labelling different time divisions within an exposure period and *k* is an index labelling different frequency ranges. For example, the modulation means 2 can configured to apply: a first effective spatial modulation pattern $\acute{Z}^1_1$ to the light 9 from a first time division in an exposure period in a first frequency range, a different second effective spatial modulation pattern $\acute{Z}^1_2$ to the light 9 from the first time division in the exposure period in a second frequency range, a different third effective spatial modulation pattern $\acute{Z}^2_1$ to the light 9 from a second time division in the exposure period in the first frequency range, and a different fourth effective spatial modulation pattern $\acute{Z}^2_2$ to the light 9 from a second time division in the exposure period in the second frequency range.

A spatial modulation pattern (code) can be configured to selectively remove information from the light 9 so that only portions of the light 9 are detected.

The different spatial patterns represent different codes that differently code the light 9. In this way, the light 9 from different time divisions and frequency ranges are code-divided at the detector 7. This code-division allows the separation of the images by time and by frequency.

It is therefore possible to capture images at a frame rate that is higher than the capture rate (1/exposure time) of the detector 7. Multiple images can be detected in a single exposure time of the detector 7. Each of the multiple images, from different time divisions, can be differently coded allowing them to be recovered from the output of the detector 7.

Each image plane in the temporal data cube (x,y,t) is modulated by a different code. Detection integrates temporally distinct image planes, but the data cube can be recovered by isolating each temporal image plane based on the distinct uncorrelated code patterns for the planes. The time-varying effective spatial modulation varies over a period of detector integration (the exposure period).

It is also possible to use the frequency division to provide two-dimensional images that have two spatial dimensions (x,y) with a third dimension. The third dimension can, for example, be a spectral dimension or, when the light 9 is provided from an OCT arrangement 17, a third spatial dimension (z). Each image plane in the spectral data cube (x,y,λ) is modulated by a different code. Detection integrates spectrally and temporally distinct image planes, but the data cube can be recovered by isolating each tempo-spectral image plane based on the distinct uncorrelated code patterns for the planes.

The different coding can, for example, be achieved using a variable spatial code. The variation in the code can be a variation over time, and optionally frequency. The modulation means can, for example, be configured to apply a different spatial modulation pattern to the received light during each time division of the exposure period and be configured to apply, subsequently, different spatial offsets to the light in dependence upon frequency.

The different spatial modulation pattern can be provided by a dynamic (time-variable) spatially coded aperture that has a time-variable pattern or has a fixed pattern and is movable. The dynamic spatially coded aperture could comprise, for example, a liquid crystal on silicon (LCOS) modulator, or a digital micromirror device (DMD) array, or a fixed pattern (passive) spatially coded aperture that is moved (translated or rotated). Movement can be performed by an electrically controlled actuator. One example of an electrically controlled actuator is a motor, for example a servo motor controlled by a processor.

In other examples, the different coding can, for example, be achieved using a fixed spatial modulation pattern (code), and a spatial offset that is different between the time divisions and is variable. The variation in the spatial offset can be a variation over time and, optionally, frequency. The time-varying spatial shift can, for example be achieved by moving an optical element configured to provides a spatial shift (an offset), when measured at the detector 7, between the spatially modulated light 11 from first time division during the exposure period and the spatially modulated light 11 from second time division during the exposure period . In at least some examples, the time-varying spatial shift can be provided by moving a dispersing means.

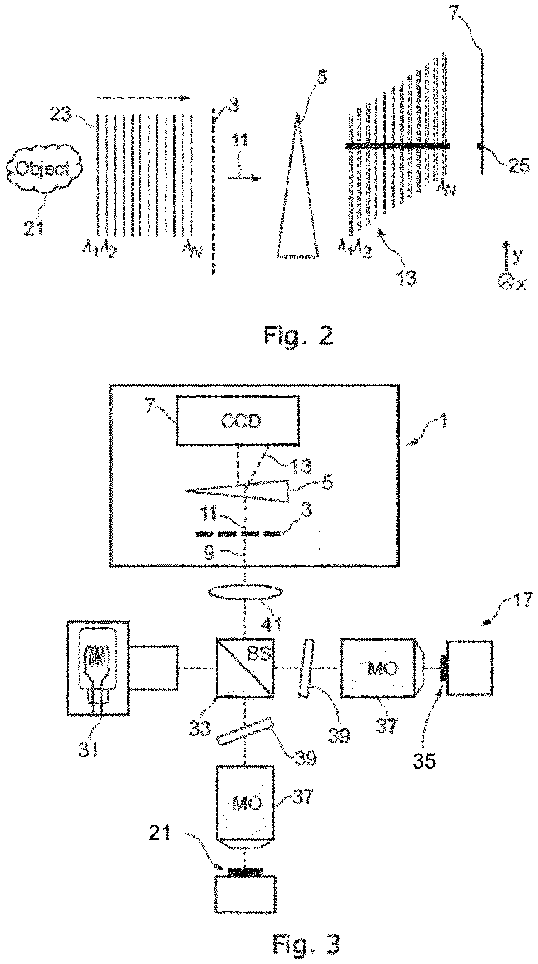

In the example illustrated in Figs. 1 and 2, the modulation means 2 comprises:

spatial modulating means 3 for spatially modulating a beam of light 9 to produce a modulated beam of light 11; dispersing means 5 for dispersing the modulated beam of light to produce a spatially modulated and dispersed beam of light; and

means 102 for causing a time-variation of the spatial modulating means 3 and/or the dispersing means 5 during the exposure period (illustrated in Fig. 1).

The spatial modulating means 3 can, in some examples, comprise one or more spatial modulators. An example of a spatial modulator 3 is a spatially coded aperture.

In the example of Fig. 1, a modulator 3 is arranged within the apparatus 1 so that when the apparatus 1 is coupled to a source of the input beam of light 9, the input beam of light 9 is incident, at least in part, upon the spatial modulator 3.

The spatial modulator 3 may selectively remove information from the input beam of light 9 so that only portions of the input beam of light 9 are detected. In some examples the spatial modulator 3 may be arranged to convert a three-dimensional signal into a coded three-dimensional signal.

The spatial modulator 3 may comprise any means which may be arranged to spatially modulate the input beam of light 9. The spatial modulation occurs over a transverse cross-sectional area of the input beam of light 9. The modulation comprises amplitude modulation that varies in dependence upon a location within the transverse cross-sectional area of the input beam of light 9.

In some examples the spatial modulator 3 comprises a spatially coded aperture. The spatially coded aperture provides for spatial modulation over a cross-sectional area of the input beam of light 9 that passes through the coded aperture. The coded aperture is coded to provide amplitude modulation that varies in dependence upon a location within the aperture. The coded aperture defines a fixed two-dimensional pattern of spatially varying transparency. The spatially coded aperture physically modulates the beam of light to a spatially compressed/sparse format.

The spatially coded aperture may comprise a non-uniform optical mask or any other suitable type of aperture that provides amplitude modulation that varies in dependence upon a location within the aperture.

The spatially coded aperture may be a two-dimensional spatially coded aperture or any other suitable type of aperture. The two-dimensional spatially coded aperture defines a two-dimensional plane. The beam of light 9 may travel in a direction normal (orthogonal) to the two-dimensional plane.

In other examples the spatial modulator 3 could comprise a liquid crystal on silicon (LCOS) modulator, a digital micromirror device (DMD) array or any other suitable type of spatial modulator 3.

The spatial modulator 3 can comprise multiple different portions that have a particular transparency. In some examples the spatial modulator 3 may comprise at least a first portion (e.g. a first plurality of first portions) having a first level of transparency to the input beam of light 9 and at least a second portion (e.g. a second plurality of second portions) having a second, different level of transparency to the input beam of light 9. In some examples the spatial modulator 3 may comprise at least multiple spatially distributed non-overlapping first portions, that are distributed over an area in two dimensions and have a first level of transparency to the input beam of light 9 and at least multiple spatially distributed non-overlapping second portions that are distributed over the area in two dimensions and have a second, different level of transparency to the input beam of light 9. In at least some examples, the spatially distributed first portions and the spatially distributed second portions do not overlap. The spatially distributed first portions and the spatially distributed second portions can be contiguous and, in some examples, the spatially distributed first portions and the spatially distributed second portions completely fill the area. The different levels of transparency may allow different levels of light to pass through the spatial modulator 3. In some examples the spatial modulator 3 may be a binary modulator 3 so that only two different absorbencies are provided by the respective portions of the spatial modulator 3. In other examples the spatial modulator 3 may be a grey-scale modulator and may comprise more than two different levels of transparency in the different portions of the spatial modulator 3.

The different portions of the spatial modulator 3 may be arranged in any suitable pattern. In some examples the respective portions of the spatial modulator 3 having different transparencies are pixelated and arranged in a pixelated pattern. The pixelated arrangement may have the respective portions of the spatial modulator 3 arranged in an array of columns and rows of pixels. In some examples, the pixels are square or rectangular.

The spatially coded aperture can comprise multiple different portions that are coded with a particular transparency, for example, the coded aperture can be pixelated and comprise multiple different portions (pixels) that are arranged as an array in rows and columns, where the pixels are coded with a particular transparency. The two-dimensional pattern of pixels (portions) that have a first transparency is different to the two-dimensional pattern of pixels (portions) that have a second transparency, different to the first transparency.

The transparency at each pixel defines a fixed two-dimensional pattern of spatially varying transparency. In some examples, the transparency at each pixel in a row defines a fixed one-dimensional pattern of spatially varying transparency that does not repeat or does not repeat within a minimum number of columns. In some examples, the transparency at each pixel in a column defines a fixed one-dimensional pattern of spatially varying transparency that does not repeat or does not repeat within a minimum number of rows. In some examples, the transparency at each pixel defines a fixed two-dimensional pattern of spatially varying transparency that has a random or pseudorandom spatial distribution. In some examples, the pixels are coded as either opaque or transparent. In other examples, the pixels are coded using grey scale.

The spatial modulation can therefore be a randomized, pixelated modulation over a two-dimensional area

The size p of the pixels when projected onto a detector 7, can be directly proportional to a size d of pixels of the detector 7.

The rows and columns of the pixels (common modulation element 32) when projected onto the detector 7, can be parallel to rows and columns of pixels of the detector 7.

The number of transparent pixels, partially transparent pixels, and opaque pixels in a spatially coded aperture can vary in different implementations of the disclosure. In some examples approximately half of the pixels of the modulator could be absorbent so that half of the incident area of the modulator acts to block the input beam of light 9 while the other half allows the incident beam of light to pass, or partially pass through in a spatially-coded format.

In some examples the different portions (e.g. pixels) of the spatial modulator 3 may be arranged in a random pattern (which encompasses pseudo random patterns) that is random in two dimensions. The random pattern may be an irregular pattern. The random pattern might not be defined or arranged in relation to any specific object. In other examples the respective portions (e.g. pixels) of the spatial modulator 3 may be arranged in a predetermined pattern.

The predetermined pattern may be selected according to the source 17 of the beam of light 9. It can for example be selected according to the object 21 or type of object that is to be imaged, for example, by an OCT arrangement 17.

In some examples the spatial modulator 3 may be fixed in position relative to the other components of the apparatus 1. In other examples the spatial modulator 3 may be arranged to be moveable relative to the other components of the apparatus 1.

In some examples the transparency of the portions of the spatial modulator 3 may be wavelength dependent. In such examples the modulation of the input beam of light 9 by the respective portions of the spatial modulator 3 will be dependent upon the wavelengths within the input beam of light 9.

The dispersing means 5 for dispersing the modulated beam of light 11 is arranged within the apparatus 1 so that the spatially modulated beam of light 11, or at least part of the spatially modulated beam of light 11, provided by the spatial modulator 3 is incident upon the dispersing means 5. Dispersion converts a spectral difference (a difference in wavelength of the light) into a spatial offset.

The dispersing means 5 can, in some examples, comprise one or more disperser elements.

The dispersing means 5 is configured to cause a wavelength dependent spatial shift of the same fixed spatially coded aperture, defined by the spatial modulator 3. In at least some examples the spatial shift is only in the plane of the aperture/beam (2D dispersion). In at least some examples, the spatial shift is only in one dimension (1D dispersion). That one dimension can be aligned with a row (or a column) of pixels in the spatially coded aperture and/or pixels of the detector 7.

The dispersing means 5 configured to disperse the modulated beam of light 11 can comprise one or more dispersing elements. The dispersing elements 5 may comprise any elements which cause different wavelengths of the modulated beam of light 11 to be dispersed by different amounts. The one or more dispersing elements 5 may comprise one or more refractive elements, for example a prism, or one or more diffractive elements, for example a grating, which can be a transmissive diffraction grating or a reflective diffraction grating or any other suitable elements.

The dispersing means 5 can be a prism or a combination of prisms. A prism is a polyhedron with two faces parallel, and with surface normals of the other faces lying in the same plane.

The or each prism can be a triangular prism. The triangular prism can have a constant triangular cross-section that has a shape of an isosceles triangle or an equilateral triangle.

The dispersing means 5 provides a spatially modulated and dispersed beam of light 13 as an output.

The detector 7 is configured to detect the dispersed beam of light 13 during exposure periods. The detector 7 is arranged within the apparatus 1 so that the spatially modulated and dispersed beam of light 13, or at least part of the spatially modulated and dispersed beam of light 13, is incident on the detector 7 for detecting the modulated and dispersed beam of light 13.

The detector can be arranged to transduce an accumulation of an incident beam of light, during an exposure period, into an electrical output signal 15. In some examples the detector 7 may comprise a charge-coupled device, complementary metal-oxide semiconductor (CMOS) sensors or any other suitable type of sensors.

In some examples the detector 7 may comprise a two-dimensional array of sensors (pixels).

The detector 7 can be configured to detect, cumulatively during an exposure period, spatially modulated light over a fixed detection area. The detector can be configured to detect, over a duration of the exposure period, spatially modulated light over the same detection area.

The beam of light 9 used during measurement can have a broad spectrum (it is broadband). It comprises light that has a broad frequency spectrum. The broadband beam of light 9 can, for example, comprise light that has wavelengths that differ by over 20nm. The broadband beam of light 9 can, for example, comprise light that has wavelengths that differ by between 20nm and 50nm.

The bandwidth of the beam of light corresponds, through the dispersing means 5, to a maximal spatial shift between spatially coded aperture patterns for different wavelengths. In at least some examples, the pattern of the spatially coded aperture does not repeat in the direction of the spatial shift for at least a distance corresponding to the maximal spatial shift.

The spatially modulated beam of light 11 is a sparse three-dimensional data cube [x, y, $\lambda$] with a two-dimensional slice [x,y] for each wavelength channel. The spatially modulated and dispersed beam of light 13 represents a skewed version of the sparse three-dimensional data cube. The skew (offset), caused by the dispersing means 5, is within the x-y plane and is proportional to wavelength. In the example illustrated in Fig. 2 it is in the y-direction only. Each spatially coded two-dimensional slice [x,y] for each wavelength channel k is shifted (offset) $y_k$. The detector 7 detects the superposition of the offset spatially coded two-dimensional slices [x,y] for each wavelength channel n. This reduces the sparse three-dimensional data cube to a two-dimensional projection in a single shot. It collapses overlapping differently spatially coded spectrograms for different channels to a single spectrogram. The different spatially coded spectrograms are incoherent.

The light 9 can, for example, arrive from any suitable source. The light 9 can, for example, comprise light that has been reflected from or that has passed through a first scene or a first object.

Where the broadband beam of light 9 has been reflected from or has passed through a scene or an object 21, then the output signal 15 provided by the detector 7 comprises information indicative of the scene or object 21. In some but not necessarily all examples, the processing means 10 uses the output signal 15 to provide a spectral image of the scene or object 21 or a three-dimensional image of the object.

Where the source 17 of the beam of light 9 is an optical coherence tomography (OCT) arrangement, the output signal 15 provided by the detector 7 comprises information indicative of the object 21 which can, for example, be a three-dimensional object imaged by the OCT arrangement 17. An OCT arrangement can be used to transform a four-dimensional spatio-temporal image [x, y, z, t] to a four-dimensional spatio-spectral-temporal image [x, y, A, t]. The spectral and temporal code-division applied by the modulation means 2 allows the separation of the image [x, y] for each data cube [x, y, $\lambda_k$, $t_l$] from the detector output by processing, for example using processor 10. Multiple images detected in a single exposure time of the detector are differently coded allowing the four-dimensional spatio-temporal image [x, y, z, t] to be recovered from the output of the detector 7. Thus, a single measurement (2D image) is detected by the detector and from this 'measurement', we can recover multiple images, which represents the 4D scenes.

In some but not necessarily all examples, the processing means 10 processes the output signal 15, for example using non-linear optimization, to produce a time-varying image. The time-varying image has two spatial dimensions (x,y) and a third dimension in space (z) or wavelength (A) forming respectively a time-varying real 3D image that has three spatial dimensions (x,y,z) or a time-varying hyperspectral (two-dimensional) image (x,y,$\lambda$). Where an optical coherence tomography (OCT) arrangement 17 provides the beam of light 9 from an object, the non-linear optimization can be used to produce a time-varying real three-dimensional image of the object 21 (a real image that has three spatial dimensions x, y, z). This produced time-varying three-dimensional image can be rendered on a display or other suitable user output device.

The processing means 10 can be a part of the apparatus 1 or, as shown in Fig. 1, separate from the apparatus 1. In some examples, the processing means 10 is remote from the apparatus 1. The processing means 10 can comprise a processor or controller and memory. The processing means 10 (or other processors) can load and use a computer program stored in a memory to perform its functions. The functions performed can, for example, comprise one or more of: controlling a time-variation of the modulation means 2, and processing an output signal 15, for example, using non-linear optimization to produce a time-varying image. The processor 10 can, therefore provide at least part of the means 102 for causing a time-variation of the spatial modulating means 3 and/or the dispersing means 5 during the exposure period.

There can therefore be provided a computer program that when run by a processor enables a processor to control:

applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector.

In, the example illustrated in Fig. 1, but not necessarily all examples, the apparatus 1 comprises means 102 for causing a time-variation of the spatial modulating means 3 and/or the dispersing means 5 during the exposure period.

The means 102 for causing a time-variation of the spatial modulating means 3 and/or the dispersing means 5 during the exposure period can for example be circuitry for controlling or enabling control of operation of the dispersing means 5 and/or the modulating means 2.

In some examples, the means 102 comprises a processor or provides a communication link from the processor 10.

In some examples, the means 102 comprises a driver for moving or adapting some or all of the dispersing means 5. In some examples, the means 102 comprises a driver for moving or adapting some or all of the modulating means 2.

In some examples, the means 102 enables time-variation, during the exposure period, of the effective spatial modulation at the detector of the received light 9. For example, it causes time-variation of a spatial modulation pattern applied during the exposure period by the spatial modulating means 3.

In some examples, the means 102 enables time-variation, during the exposure period, of the spatial modulator 3 (e.g. the spatially coded aperture) such that a different spatial modulation (e.g. a different spatially coded aperture) is provided for the light 9 at each time division within an exposure period. The spatial modulator 3 (e.g. spatially coded aperture) is a dynamically variable spatial modulator (e.g. a dynamically variable spatially coded aperture) and can be controlled to change it spatial modulation (e.g. spatial coding). In these examples the spatial modulator 3 (e.g. the spatially coded aperture) could comprise a liquid crystal on silicon (LCOS) modulator, a digital micromirror device (DMD) array or any other suitable type of spatial modulator 3.

In some examples, the means 102 enables time-variation, during the exposure period, of a position of a fixed spatial modulator 3 (e.g. fixed spatially coded aperture) such that a different offset of a fixed spatially coded aperture is provided for the light 9 at each time division within an exposure period.

In some examples, the means 102 enables time-variation, during the exposure period, of a position of the dispersion element 5. For example, the dispersion element 5 can be laterally shifted and/or rotated.

In some but not necessarily all examples, the dispersing means 5 provides dispersion in a dispersion direction. The dispersion direction is the direction in which light is shifted spatially by dispersion means 5. A relative movement of the dispersing means 5 and the detector 7 causes relative movement in the dispersion direction. The dispersion direction can be aligned with rows or columns of pixels in the detector 7 and/or the dispersion direction can be aligned with rows or columns of modulating pixels in the spatial modulating means 3.

Fig. 2 shows the compressive sensing principle of examples of the disclosure. In the example of Fig. 2, the object 21 reflects broadband light which has been directed onto the object 21. Different wavelengths of the incident light are reflected differently depending upon the internal structure of the object 21. This provides a plurality of spatial images 23. Each of the spatial images 23 corresponds to a different wavelength of light $\lambda_k$ ($\lambda_1$ to $\lambda_N$). The different spatial images 23 therefore comprise information about the internal structure of the object 21. The different spatial images 23 define a three-dimensional signal $[x, y, \lambda_k]$. The different spatial images 23 vary over time t (within an exposure period of the detector) and define a four-dimensional signal $[x, y, \lambda_k, t_l]$.

The reflected beam of light 9 is a four-dimensional data cube $X=[x, y, \lambda_k, t_l]$ with a two-dimensional slice $[x,y]$, a spatial image 23, for each frequency range (wavelength channel $\lambda_k$) at each time division $t_l$.

In the example of Fig. 2 the spatial modulator 3 comprises a time-variable two-dimensional spatially coded aperture. Other types of modulator 3 may be used in other examples of the disclosure, for example as previously described.

The two-dimensional spatial images (x, y) 23 in the input beam of light 9 are modulated by the spatially coded aperture to produce a spatially modulated beam of light 11.

The spatially modulated beam of light 11 is a sparse four-dimensional data cube $[x, y, \lambda_k, t_l]$ with a two-dimensional slice $[x,y]$ for each wavelength channel $\lambda_k$ coded by the spatially coded aperture that has variable transparency in the x-y plane that changes with each time division $t_l$.

Let the four-dimensional data cube $[x, y, \lambda_k, t_l]$ be represented by X, where

$$X \in \mathbb{R}^{N_x \times N_y \times N_\lambda \times N_t}$$

Let the dynamic time-varying spatially coded aperture be represented by mask M* where

$$M^* \in \mathbb{R}^{N_x \times N_y \times N_t}$$

Let the tempo-spatially modulated data cube after the mask M* be represented by Z where

$$Z \in \mathbb{R}^{N_x \times N_y \times N_\lambda \times N_t}$$

In this example, but not necessarily all examples, let us assume a one-to-one correspondence between the [i,j] space at the detector 7 where $(i, j) \in \mathbb{R}^{N_x \times N_y}$ and the [x,y] space at the coded aperture where $(x, y) \in \mathbb{R}^{N_x \times N_y}$.

Then

$$Z_k^l(i,j) = X_k^l(i,j) \odot M^l(i,j),$$

$$\forall k = 1, \dots, N_\lambda, \forall l = 1, \dots, N_t,$$

⊙ *denotes elementwise product in (i,j) dimension*

The spatially modulated beam of light 11 provided by the spatial modulator 3 is then spread by the dispersing element 5. In the example of Fig. 2 the dispersing element 5 comprises a triangular prism. Other types of dispersing element 5 could be used in other examples of the disclosure, as previously described. In this example, the dispersing element 5 refracts the modulated beam of light 11 to spatially spread the modulated beam of light in the y-direction only. Different wavelengths of the spatial images 23 are spread by a different amount in the y-direction as shown schematically in Fig. 2. The distance by which a spatial image 23 is spread by the dispersing element 5 is dependent upon (e.g. proportional to) the wavelength of the spatial image 23.

The spatially modulated and dispersed beam of light 13 represents a skewed version of sparse multi-dimensional data cube. The skew (offset), caused by the dispersing means 5, is within the x-y plane and is proportional to wavelength. In the example illustrated in Fig. 2 it is in the y-direction only. Each spatially coded two-dimensional slice [x,y] for each wavelength channel k is shifted (offset) $y_k$.

Let the tempo-spatially modulated data cube after the mask M* and disperser be represented by $\acute{Z}$ where

$$\acute{Z} \in \mathbb{R}^{N_x \times (N_y + N_\lambda - 1) \times N_\lambda \times N_t}$$

$$\acute{Z}_k^l(i,j) = Z_k^l(i, j + g(\lambda_k - \lambda_c))$$

The dispersing means 5 causes a spatial shift $g(\lambda_k - \lambda_c)$ in the y-direction (where $\lambda_k - \lambda_c$ is the spectral shift of the wavelength $\lambda_k$ from a central wavelength $\lambda_c$) then:

$$M\big(i, j + g(\lambda_k - \lambda_c)\big) = M^*(i,j)$$

The spatially modulated and dispersed beam of light 13 is then incident upon the detector 7. The detector 7 comprises a plurality of pixels 25. Only one pixel 25 is shown for clarity in Fig. 2. The plurality of pixels 25 may be arranged in any suitable array. In the example of Fig. 2 the plurality of pixels 25 may be arranged in a matrix array comprising rows and columns. Each pixel 25 detects, during an exposure period, the summation of the modulated and dispersed beam of light 13 for each of the different wavelengths $\lambda_1$ to $\lambda_N$ for the area covered by the pixel 25.

As the different wavelengths $\lambda_1$ to $\lambda_N$ in the dispersed beam of light 13 are shifted by different amounts the different wavelengths $\lambda_1$ to $\lambda_N$ that are incident on a given pixel of the detector 7 have passed though different (incoherent) portions of the spatial modulator 3. This means that the different wavelengths $\lambda_1$ to $\lambda_N$ that are incident on a given pixel 25 of the detector 7 may be spatially modulated by different amounts.

The detector 7 detects the superposition of the offset spatially coded spatio-temporal slices [x, y $t_l$] for each wavelength channel $\lambda_k$ over the exposure period. This reduces the sparse four-dimensional data cube to a compressed two-dimensional projection in a single shot. It collapses overlapping differently spatially coded spectrograms for different channels, at different times to a single spectrogram.

$$Y = \sum_{l=1}^{N_t} \sum_{k=1}^{N_\lambda} \acute{Z}_k^l + E$$

$$E \in \mathbb{R}^{N_x \times (N_y + N_\lambda - 1)} \qquad measurement\ noise$$

Thus, a different effective spatial modulation pattern $\acute{Z}_k^l$ can be applied for each different combination (*l* , *k*) where *l* is an index labelling different time divisions within an exposure period and *k* is an index labelling different frequency ranges.

The spectrogram Y can be re-expressed as

$$Y = \sum_{l=1}^{N_t} \sum_{k=1}^{N_\lambda} \acute{X}_k^l \odot \acute{M}_k^l + E$$

$$\acute{X} \in \mathbb{R}^{N_x \times (N_y + N_\lambda - 1) \times N_\lambda \times N_t}$$

$$\acute{X}_k^l(i,j) = X_k^l(i, j + g(\lambda_k - \lambda_c))$$

$$\acute{M} \in \mathbb{R}^{N_x \times (N_y + N_\lambda - 1) \times N_\lambda \times N_t}$$

$$\acute{M}_k^l(i,j) = M^l(i, j + g(\lambda_k - \lambda_c))$$

This allows the measurement Y obtained by each pixel 25 to be written in vectorized form as

$$y = \Phi x + e$$

Where

$$y = vec(Y) \in \mathbb{R}^{N_x(N_y + N_\lambda - 1)}$$

*y* is a vectorized version of the measurement obtained by each pixel 25,

$$x = vec(\acute{X}) \in \mathbb{R}^{N_x(N_y + N_\lambda - 1)N_\lambda N_t}$$

*x* is the stacked vector of the three-dimensional input beam of light

$$e = vec(E) \in \mathbb{R}^{N_x(N_y + N_\lambda - 1)}$$

*e* is a vectorized version of the noise

$$\Phi \in \mathbb{R}^{(N_x(N_y + N_\lambda - 1)) \times (N_x(N_y + N_\lambda - 1)N_\lambda N_t)}$$

*Φ* is the sensing matrix and can be represented by diagonal matrices:

$$\Phi = \left\{ \left[ D_{1,1}, D_{1,2} \dots, D_{1,N_\lambda} \right], \left[ D_{2,1}, D_{2,2} \dots, D_{2,N_\lambda} \right], \dots, \left[ D_{N_t,1}, D_{N_t,2} \dots, D_{N_t,N_\lambda} \right] \right\},$$

$$D_{l,k} = diag(vec(\acute{M}_k^l))$$

The series of time-varying images [x, y, λ, t] can therefore be obtained by solving

$$\hat{x} = argmin_x \|y - \Phi x\|_2^2 + \tau R(x)$$

where $R(x)$ denotes the regularizer imposed on the image, and $\tau$ balances the two terms in equations. The regularizer R can, for example, be based upon total variation of the three-dimensional image.

The series of time-varying images in three spatial dimensions [x, y, z, t] can therefore be obtained by solving

$$r = \arg\min_{r} ||y - \Phi Fr||^2 + \tau R(r)$$

where

$$x = Fr$$

where F is a Fourier transform.

And where $R(r)$ denotes the regularizer imposed on the image, and $\tau$ balances the two terms in equations. The regularizer R can, for example, be based upon total variation of the three-dimensional image.

Any suitable compressive sensing inversion algorithms may be used by processing means 10 to solve the equation to obtain the desired image. For example, non-linear optimization can be used to produce a time-varying three-dimensional image of the object. The time-varying image has two spatial dimensions (x,y) and a third dimension in space (z) or wavelength (A) forming respectively a time-variable real image that has three spatial dimensions (x,y,z,t) or a time-variable hyperspectral (two-dimensional) image (x,y,A,t). Where an optical coherence tomography (OCT) arrangement 17 provides the beam of light 9 from an object, the non-linear optimization can be used to produce a time-varying real three-dimensional image of the object (a time series of real images that have three spatial dimensions (x, y, z)).

The sparsity of the transfer function $\Phi$ that represents the modulating means 2 e.g. the combination of the spatial modulating means 3 and the dispersing means 5 causes information compression.

The compression is in the time domain and the spectral domain. The apparatus 1 can therefore be described as performing temporal-spectral compressive sensing.

Figs. 1 and 2 therefore illustrate examples of a method comprising:

applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector; and
detecting, during the exposure period, the spatially modulated light.

Fig. 3 illustrates an example in which the source 17 of the broadband beam of light 9 provided to the apparatus 1 is an optical coherence tomography arrangement 17.

Optical coherence tomography (OCT) is also called low coherence light interferometry. A double path interferometer 17 comprising a sample path for an object 21 and a reference path. In this example, the double path interferometer arrangement 17 is used in a Michelson configuration to create interference between an optical beam reflected from an object 21 in the sample path and a reference optical beam in the reference path. The above described apparatus 1 can be used to detect the interference.

A double path interferometer, for example a Michelson interferometer, uses a beam splitter 33, to split light from a light source 31 into two beams of the same bandwidth that travel at 90 degrees to each other along different paths- a sample (object) path and a reference path . In the Michelson interferometer, each of those light beams is reflected back toward the beam splitter 33 which then combines their amplitudes using the superposition principle. The resulting interference pattern is directed to the detector 7 via spatial spatial modulating means 3 and dispersing means 5.

In this example, a light beam is reflected back from the object 21 and the other reference light beam is reflected back from a mirror 35.

The arrangement 17 is a temporal-spectral compressive sensing optical coherence tomography (TS-CS-OCT) arrangement that uses a modulating means 2 (e.g. spatial modulating means 3 before dispersing means 5) on the interference signal before detection of a spectrogram of overlapping differently spatially coded spectrograms for different wavelength channels.

The detector 7 can detect the different wavelengths simultaneously so that all the information is recorded in single image (one-shot operation). The apparatus 1 therefore enables $N_\lambda$ channel signals to be recovered from a single measurement. The detector 7 produces, in a single shot, without spatial or spectral scanning, spectral domain information 15 that can be used to produce a three-dimensional image of the object 21.

In this example there is full field illumination of the object 21 by a light beam over an area. A reflected beam of light from the object 21 provides full field illumination of a spatially coded aperture 9 over an area. The spatially coded light provides full-field illumination of a disperser element 5 over an area. The dispersed, spatially coded light 13 provides full field illumination of the detector 7 over an area that corresponds to an array of $N_x$ by $N_y$ pixels (sensels) in the detector 7.

The OCT arrangement 17 comprises a light source 31, a beam splitter 33, a static reference mirror 35, one or more microscope objectives 37, one or more compensators 39, and one or more focusing elements 41. The OCT arrangement 17 is a spectral domain arrangement. It uses a light source 31 of a fixed broad spectrum.

In examples of the disclosure the light source 31 used for measurement is a broadband light source which provides light having a range of wavelengths. The wavelength of the light that is used may depend on the type of object 21 that is to be imaged or any other suitable factor. In some examples the light used may be infrared light. in some examples the wavelength of the light used may have a range of wavelengths between 400nm and 1500nm. The "centre wavelength" can be for example at 830nm with a frequency range of 810nm to 850nm for a 40nm bandwidth or the "centre wavelength" can be 1300nm with a frequency range of 1280nm to 1320nm for a 40nm bandwidth or the "centre wavelength" can be 1500nm with a frequency range of 1480nm to 1520nm for a 40nm bandwidth. Other centre wavelengths and other bandwidths are possible.

The output light beam from the light source 31 is incident on the beam splitter 33. The beam splitter 33 may comprise a prism, a half silvered mirror or any other suitable component.

In the OCT arrangement 17 half of the split beam provides the reference beam and is provided to the static reference mirror 33. A microscope objective 37 and a compensator 39 are provided between the beam splitter 33 and the static reference mirror 35. The microscope objective 37 may comprise any means which may be arranged to focus the beam of light. In some examples the microscope objective 37 may comprise one or more lenses or any other suitable optical elements. The compensator 39 may comprise a compensator plate or any other suitable compensating element. In the example of Fig. 3 the compensator 39 comprises a decoupling compensator polarizer.

The other half of the split beam provides the object beam and is provided to the object 21. The object 21 may be arranged to be moved along the z axis, but not during imaging. This axis may enable the focussing of the three-dimensional images provided by the OCT arrangement 17. In the example of Fig. 3 the object 21 is provided on a motorised arrangement so as to enable movement along the z axis between one-shot measurements. In other examples a manual arrangement, or any other suitable type of arrangement, could be used.

A microscope objective 37 and a compensator 39 are provided between the beam splitter 33 and the object 21. The microscope objective 37 may comprise any means which may be arranged to focus the beam of light. In some examples the microscope objective 37 may comprise one or more lenses or any other suitable optical elements. The compensator 39 may comprise a compensator plate or any other suitable compensating element. In the example of Fig. 3 the compensator 39 comprises a decoupling compensator polarizer.

The different wavelengths of the light provide coherence of the object beam and the reference beam at different optical path lengths. Therefore, the different wavelengths of light provide information about different depths within the object 21. Different features within the object 21 reflect the incident light by different amounts. The interference between the reflected object beam and the reflected reference beam therefore provides information about the features within the object 21.

As the different frequencies of light provide information about different depths within the object 21 this enables three-dimensional imaging of the object 21. The three-dimensional imaging 21 may enable different features at different depths within the object to be identified and/or analysed. This ensures that the information obtained in the examples of the disclosure comprises information about the internal structure of an object 21 and not just information about the surface of the object 21.

The OCT arrangement 17 also comprises one or more focusing elements 41. The focussing element 41 may comprise a lens or any other suitable means for focusing a beam of light. The focusing element 41 is arranged to focus the input beam of light 9 into the apparatus 1 for detecting.

In the example of Fig. 3 the apparatus 1 comprises a spatial modulating means 3, a dispersing means 5 and a detector 7 as previously described. The input beam of light 9 provided to the apparatus 1 is from the OCT arrangement 17.

Referring back to Fig. 1, the means 102 can be provided by a controller. Implementation of a controller may be as controller circuitry. The controller may be implemented in hardware alone, have certain aspects in software including firmware alone or can be a combination of hardware and software (including firmware).

The controller may be implemented using instructions that enable hardware functionality, for example, by using executable instructions of a computer program 102 in a general-purpose or special-purpose processor that may be stored on a computer readable storage medium (disk, memory etc) to be executed by such a processor.

The processor can be configured to read from and write to a memory. The processor may also comprise an output interface via which data and/or commands are output by the processor and an input interface via which data and/or commands are input to the processor.

The memory can store a computer program 103 comprising computer program instructions (computer program code) that controls the operation of the apparatus 1 when loaded into the processor. The computer program instructions, of the computer program 103, provide the logic and routines that enables the apparatus to perform the methods described.

The processor by reading the memory is able to load and execute the computer program 103.

The apparatus 1 can therefore comprise:

at least one processor; and
at least one memory including computer program code
the at least one memory and the computer program code configured to, with the at least one processor, cause the apparatus at least to perform:
applying multiple different effective spatial modulations to received light during an exposure period of a detector, wherein a different effective spatial modulation is applied to received light in dependence upon a time during the exposure period of the detector and a frequency of the light, to produce spatially modulated light for detection by the detector.

The computer program 103 may arrive at the apparatus 1 via any suitable delivery mechanism. The delivery mechanism may be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 103. The delivery mechanism may be a signal configured to reliably transfer the computer program 103. The apparatus 1 may propagate or transmit the computer program 103 as a computer data signal.

The computer program instructions may be comprised in a computer program, a non-transitory computer readable medium, a computer program product, a machine readable medium. In some but not necessarily all examples, the computer program instructions may be distributed over more than one computer program.

A memory can be implemented as one or more separate components/circuitry some or all of which may be integrated/removable and/or may provide permanent/semi-permanent/ dynamic/cached storage.

A processor can be implemented as one or more separate components/circuitry some or all of which may be integrated/removable. The processor can be a single core or multi-core processor.

References to 'computer-readable storage medium', 'computer program product', 'tangibly embodied computer program' etc. or a 'controller', 'computer', 'processor' etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term 'circuitry' may refer to one or more or all of the following:

(a) hardware-only circuitry implementations (such as implementations in only analog and/or digital circuitry) and
(b) combinations of hardware circuits and software, such as (as applicable):

(i) a combination of analog and/or digital hardware circuit(s) with software/firmware and
(ii) any portions of hardware processor(s) with software (including digital signal processor(s)), software, and memory(ies) that work together to cause an apparatus, such as a mobile phone or server, to perform various functions and

(c) hardware circuit(s) and or processor(s), such as a microprocessor(s) or a portion of a microprocessor(s), that requires software (e.g. firmware) for operation, but the software may not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

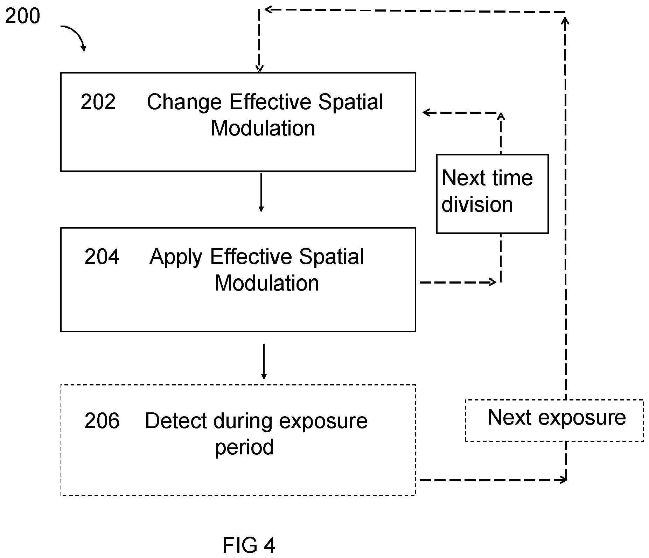

FIG 4 illustrates an example of a method 200.

The method comprises at blocks 202, 204 applying multiple different effective spatial modulations to received light, during an exposure period of a detector, wherein a different effective spatial modulation is applied to received light in dependence upon a time during the exposure period of the detector and a frequency of the light, to produce spatially modulated light for detection by the detector; and

optionally, ay block 206, detecting, during the exposure period, the spatially modulated light.

In at least some examples a computer program when run by a processor enables the processor to control blocks 202, 204. The computer program is configured to control application of multiple different effective spatial modulations to received light during an exposure period of a detector, wherein a different effective spatial modulation is applied to received light in dependence upon a time during the exposure period of the detector and a frequency of the light, to produce spatially modulated light for detection by the detector.

At block 202, an effective spatial modulation that is for application to received light is changed in dependence upon a time during a current exposure period of the detector (and a frequency of the light).

At block 204, the changed effective spatial modulation is applied to received light to produce spatially modulated light for detection by the detector.

The method then returns to block 202 to change the effective spatial modulation in dependence upon a new time (new time division) during the current exposure period of the detector.

Where a structural feature has been described, it may be replaced by means for performing one or more of the functions of the structural feature whether that function or those functions are explicitly or implicitly described.

Optionally, the method 200 can be repeated for multiple different exposure periods of the detector.

The above described examples find application as enabling components of: automotive systems; telecommunication systems; electronic systems including consumer electronic products; distributed computing systems; media systems for generating or rendering media content including audio, visual and audio visual content and mixed, mediated, virtual and/or augmented reality; personal systems including personal health systems or personal fitness systems; navigation systems; user interfaces also known as human machine interfaces; networks including cellular, non-cellular, and optical networks; ad-hoc networks; the internet; the internet of things; virtualized networks; and related software and services.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term 'example' or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

**Claims**

1. An apparatus comprising:

   a detector configured to detect, cumulatively during an exposure period, spatially modulated light; and
   modulation means for applying multiple different effective spatial modulations to received light, during the exposure period, wherein a different effective spatial modulation is applied to received light in dependence upon a time during the exposure period and a frequency of the light, to produce spatially modulated light for detection by the detector.

**2.** An apparatus as claimed in claim 1, wherein the different effective spatial modulation is configured to cause modulation by different spatial modulation patterns, at the detector, of the received light, wherein different spatial patterns are used for different time divisions within the exposure period and wherein, within each time division, different spatial patterns are used for distinct frequency ranges.

**3.** An apparatus as claimed in claim 1 or 2, wherein the modulation means is configured to apply a different spatial modulation pattern to the received light during each time division of the exposure period and is configured to apply, subsequently, different spatial offsets to the light in dependence upon frequency.

**4.** An apparatus as claimed in any preceding claim wherein the modulation means comprises:

spatial modulating means for spatially modulating a beam of light to produce a modulated beam of light;
dispersing means for dispersing the modulated beam of light to produce a spatially modulated and dispersed beam of light; and
means for causing a time-variation of the spatial modulating means and/or a time-variation of the dispersing means during the exposure period.

**5.** An apparatus as claimed in claim 4, wherein the means for causing a time-variation of the spatial modulating means during the exposure period causes a time-variation of a spatial modulation pattern applied during the exposure period by the spatial modulating means.

**6.** An apparatus as claimed in claim 4 or 5, wherein the spatial modulating means applies a spatial modulation to the beam of light to produce the modulated beam of light, and wherein the spatial modulation is randomized, pixelated modulation over a two-dimensional area.

**7.** An apparatus as claimed in any of claim 4, 5 or 6, wherein the spatial modulating means applies a spatial modulation to the beam of light to produce the modulated beam of light, and wherein the spatial modulation is pixelated modulation over a two-dimensional area, and the pixelated modulation comprises pixels arranged in rows and columns that are parallel to rows and columns of pixels of the detector when projected onto the detector.

**8.** An apparatus as claimed in any of claims 4 to 7, wherein the spatial modulating means comprises a two-dimensional spatially coded aperture comprising at least a first plurality of portions, having a first transparency, and at least a second plurality of portions, having a second different transparency, wherein the first plurality of portions and the second plurality of portions are spatially distributed in two dimensions.

**9.** An apparatus as claimed in any of claims 4 to 8, wherein the spatial modulating means comprises a spatially coded aperture that has a time variable pattern or has a fixed pattern and is movable.

**10.** An apparatus as claimed in any of claims 4 to 9, wherein the means for causing a time-variation of the dispersing means during the exposure period causes a time variation of a position of the dispersion means during the exposure period.

**11.** An apparatus as claimed in any preceding claim, wherein the dispersing means comprises one or more refractive elements or one or more diffractive elements.

**12.** A system comprising the apparatus as claimed in any preceding claim, wherein the system or apparatus further comprises processing means for processing output of the detector to obtain a time-varying image of at least part of an object wherein an input beam of light for spatial modulation and detection by the apparatus comprises light reflected from the object; and
means for providing a time-varying image of at least part of the object from the electrical output signals.

**13.** A system as claimed in claim 12, wherein the input beam of light for spatial modulation and detection by the apparatus is obtained from an optical coherence tomography arrangement and the provided time-varying image is a time-varying real three-dimensional image of the object that has three spatial dimensions.

**14.** A method comprising:

applying multiple different effective spatial modulations to received light, during an exposure period of a detector, wherein a different effective spatial modulation is applied to received light in dependence upon a time during the exposure period of the detector and a frequency of the light, to produce spatially modulated light for detection by the detector; and

detecting, during the exposure period, the spatially modulated light.

15. A computer program that when run by a processor enables the processor to control:

applying multiple different effective spatial modulations to received light, during an exposure period of a detector, wherein a different effective spatial modulation is applied to received light in dependence upon a time during the exposure period of the detector and a frequency of the light, to produce spatially modulated light for detection by the detector.

FIG. 1

Fig. 2

Fig. 3

200

202    Change Effective Spatial
        Modulation

Next time
division

204    Apply Effective Spatial
        Modulation

206    Detect during exposure
        period

Next exposure

FIG 4

Europäisches
Patentamt

European
Patent Office

Office européen
des brevets

# EUROPEAN SEARCH REPORT

Application Number

EP 20 17 9849

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/366763 A1 (LIN JOSEPH HSUHUAN [US] ET AL) 21 December 2017 (2017-12-21)<br>* paragraph [0039] - paragraph [0043]; figures 1-10 *<br>* paragraph [0067] - paragraph [0073] *<br>* paragraph [0054] - paragraph [0060] *<br>----- | 1-15 | INV.<br>H04B10/516<br>G01B9/02<br>G01J3/02 |
| X | US 2015/116563 A1 (HERMAN MATTHEW A [US] ET AL) 30 April 2015 (2015-04-30)<br>* paragraph [0002]; figures 2-5 *<br>* paragraph [0012] - paragraph [0015] *<br>* paragraph [0047] - paragraph [0060] *<br>* paragraph [0109] - paragraph [0116] *<br>----- | 1-15 | |
| A | EP 3 477 246 A1 (NOKIA TECHNOLOGIES OY [FI]) 1 May 2019 (2019-05-01)<br>* the whole document *<br>----- | 1-15 | |
| A | EP 3 653 987 A1 (NOKIA TECHNOLOGIES OY [FI]) 20 May 2020 (2020-05-20)<br>* the whole document *<br>----- | 1-15 | TECHNICAL FIELDS SEARCHED (IPC)<br><br>H04B<br>G01B<br>G01J |

The present search report has been drawn up for all claims

| Place of search | Date of completion of the search | Examiner |
|---|---|---|
| Munich | 25 November 2020 | Gäde, Sebastian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 9849

This annex lists the patent family members relating to the patent documents cited in the above-mentioned European search report.
The members are as contained in the European Patent Office EDP file on
The European Patent Office is in no way liable for these particulars which are merely given for the purpose of information.

25-11-2020

| Patent document cited in search report | | Publication date | Patent family member(s) | | Publication date |
|---|---|---|---|---|---|
| US 2017366763 | A1 | 21-12-2017 | US 2017366763 A1<br>WO 2018067212 A2 | | 21-12-2017<br>12-04-2018 |
| US 2015116563 | A1 | 30-04-2015 | NONE | | |
| EP 3477246 | A1 | 01-05-2019 | CN 111344533 A<br>EP 3477246 A1<br>KR 20200079279 A<br>WO 2019081807 A1 | | 26-06-2020<br>01-05-2019<br>02-07-2020<br>02-05-2019 |
| EP 3653987 | A1 | 20-05-2020 | EP 3653987 A1<br>WO 2020100064 A1 | | 20-05-2020<br>22-05-2020 |

EPO FORM P0459

For more details about this annex : see Official Journal of the European Patent Office, No. 12/82